# EUROPÄISCHE PATENTANMELDUNG

(11) **EP 1 559 372 A1**
(43) Veröffentlichungstag der Anmeldung: **03.08.2005**
(21) Anmeldenummer: 05405041.4
(22) Anmeldetag: 27.01.2005
(51) Int. Cl.: A61B 5/22

(54) **Körperaktivitätsmonitor**

(30) Priorität: 27.01.2004 CH 1072004
(71) Anmelder: Improve Gmbh, 4501 Solothurn (CH)
(72) Erfinder: Buchholz, Horst, 3147 Mittelhäusern (CH); Mäder, Urs, 2532 Magglingen (CH)
(74) Vertreter: Seehof, Michel

(57) **Zusammenfassung**

Die Erfindung betrifft einen Körperaktivitätsmonitor mit Mitteln (2) zur Eingabe bekannter Körperdaten wie Grösse, Geschlecht und Gewicht eines Benutzers des Monitors, Mitteln (9, 11) zur Messung mindestens einer als Aktivitätsindikator dienenden, von der Körperaktivität des Benutzers abhängigen physiologischen Grösse, beispielsweise der Herzfrequenz und/oder einer von der Beschleunigung abgeleiteten Bewegungsintensität, einer Eichungsprozedur (15) zur Ermittlung der benutzerspezifischen Sensibilität der Aktivitätsindikatoren und deren Speicherung als Teil der Körperdaten des Benutzers, und mit einer Energieverbrauchs-Funktion (20), die es erlaubt, anhand der genannten Körperdaten und der Werte der Aktivitätsindikatoren den Energieverbrauch des Benutzers zu ermitteln.

## Beschreibung

Die vorliegende Erfindung betrifft einen Körperaktivitätsmonitor gemäss dem Oberbegriff von Anspruch 1.

Ein Körperaktivitätsmonitor, hiernach auch kurz "Monitor" genannt, ist ein Gerät, das es erlaubt, den von der Körperaktivität abhängigen Energieverbrauch des Körpers eines Benutzers zu erfassen und zu überwachen. Eine ausgewogene Energiebilanz, das heisst das Gleichgewicht von Nahrungs- und somit Energieaufnahme auf der einen Seite und Energieverbrauch durch Stoffwechsel auf der anderen Seite, ist eine wesentliche Voraussetzung für das körperliche Wohlbefinden. In der modernen, durch Technologie und Konsum geprägten Gesellschaft bewegen sich viele Leute nur ungenügend, was insbesondere bei der gleichzeitigen Tendenz zu übermässigem Nahrungskonsum dazu führt, dass mehr Energie aufgenommen als verbraucht wird. Die Folge davon ist Übergewicht, das zu gesundheitlichen Problemen sowohl körperlicher als vielfach auch psychischer Art führt.

Ist die Energiebilanz positiv so nimmt der Körper an Gewicht zu, ist sie negativ nimmt er ab. Aus dieser bekannten Tatsache ergeben sich die bekannten Ratschläge an übergewichtige Personen, sie sollen sich mehr bewegen und weniger kalorienreich essen. In der Praxis erweist sich das jedoch regelmässig als schwierig, da nur dauerhafte Disziplin den gewünschten Erfolg verspricht und es dafür häufig an der nötigen Motivation fehlt. Mit einem Monitor, der den Energieverbrauch laufend überwacht und dem Benutzer so eine Selbstkontrolle seiner Energiebilanz ermöglicht, kann diese Motivation entscheidend gestärkt werden.

Bereits bekannte Monitore dieser Art verfügen über Mittel zur Eingabe von Körperdaten wie Alter, Gewicht und Geschlecht, eine Messvorrichtung zur Erfassung einer von der Bewegungsaktivität abhängigen physiologischen Grösse, zum Beispiel der Herzfrequenz oder der vertikalen Körperbeschleunigung, und eine Datenverarbeitungs-Einheit, die anhand dieser Daten durch die Auswertung einer in der Regel empirisch entwickelten Energieverbrauchs-Funktion den Energieverbrauch ermittelt.

Es ist eine Aufgabe der Erfindung, einen Körperaktivitätsmonitor zu schaffen, der im Vergleich zu den bekannten Monitoren eine genauere Ermittlung des Energieverbrauchs ermöglicht.

Diese Aufgabe wird durch einen Monitor mit den Merkmalen von Anspruch 1 gelöst. Weitere Aufgaben und bevorzugte Ausführungsformen sind in den abhängigen Ansprüchen angegeben.

Der Energieverbrauch des menschlichen Körpers setzt sich zusammen aus der Summe von Ruheumsatz und Aktivitätsumsatz. Der Ruheumsatz ist von der köperlichen Konstitution, d.h. von Körperdaten wie Alter, Gewicht und Geschlecht abhängig und im Wesentlichen definiert. Hingegen ist der Aktivitätsbezogene Energieumsatz nicht nur von der körperlichen Konstitution sondern primär von der körperlichen Aktivität abhängig. Zur Berechnung dieses Energieverbrauchs wird daher nebst den genannten Körperdaten auch eine Information zur Körperaktivität benötigt.

Ein erfindungsgemässer Monitor verfügt über Mittel zur Eingabe und Speicherung von Körperdaten des Benutzers und Mittel zur Messung einer oder mehrerer von der Körperaktivität des Benutzers abhängiger physiologischer Grössen, die im Folgenden allgemein als Aktivitätsindikatoren bezeichnet werden. Die Herzfrequenz oder eine von der vertikalen Körperbeschleunigung eines Benutzers, d.h. von der Beschleunigung in Längsrichtung des gestreckten Körpers, abgeleitete Grössen haben sich als für die Ermittlung des Energieverbrauchs grundsätzlich geeignete Aktivitätsindikatoren erwiesen. Überraschend hat sich gezeigt, dass sich ausserdem von der Körperbeschleunigung in Laufrichtung, d.h. von der horizontal vorwärts- bzw. rückwärts gerichteten Beschleunigung des senkrecht stehenden, aufrechten Körpers, eine in Kombination mit der Herzfrequenz und/oder der vertikalen Beschleunigung für die Körperaktivität repräsentative Grösse ableiten lässt. An Stelle der genannten, oder in Kombination damit, können aber auch andere Aktivitätsindikatoren, insbesondere von in anderen Richtungen gemessenen Beschleunigungswerten abgeleitete Grössen, für die Berechnung des Energieverbrauchs verwendet werden.

Die zum gleichen Zeitpunkt gemessenen Werte der Aktivitätsindikatoren werden im Folgenden allgemein als Indikatorentupel bezeichnet. Die Anzahl der in einem Tupel enthaltenen Werte entspricht der Anzahl der Aktivitätsindikatoren.

Die bei einer bestimmten Aktivität gemessenen Aktivitätsindikatoren sind nicht nur von dieser Aktivität (z.B. langsames Gehen) abhängig, sondern auch von der körperlichen Konstitution der die Aktivität ausführenden Person. Beispielsweise können verschiedene Personen bei derselben körperlichen Aktivität unterschiedliche Herzfrequenzen haben, dasselbe gilt jedoch auch für andere Aktivitätsindikatoren. Die Sensibilität der Aktivitätsindikatoren ist also vom Benutzer des Monitors abhängig, sie ist benutzerspezifisch. Aus den Werten der Aktivitätsindikatoren bei einer oder mehreren bestimmten Referenzaktivitäten eines Benutzers ergeben sich Informationen zu dieser benutzerspezifischen Sensibilität der Aktivitätsindikatoren und die Berücksichtigung dieser Informationen ermöglicht eine genauere Bestimmung des Energieverbrauchs des Benutzers.

Durch die Bestimmung einer Information zur benutzerspezifischen Sensibilität der Aktivitätsindikatoren und die Berücksichtigung dieser Information in der Energieverbrauchs-Funktion wird der Monitor auf einen bestimmten Benutzer geeicht. Dafür verfügt der Monitor über eine Eichungseinheit, die dafür vorgesehen ist, bei einer oder mehreren Referenzaktivitäten des Benutzers die Aktivitätsindikatoren zu messen, von diesen (im Folgenden auch als Referenztupel bezeichneten) Indikatorentupeln eine für die benutzerspezifische Sensibilität der Aktivitätsindikatoren bezeichnende Indikatorenreferenz abzuleiten und diese zu speichern. Die Indikatorenreferenz ist ein Referenzobjekt, das eine Information zur benutzerspezifischen Sensibilität der Aktivitätsindikatoren enthält. Es kann sich dabei entweder um die Referenztupel selbst oder um ein anderes von diesen abgeleitetes Objekt handeln. Die Indikatorenreferenz ist Teil der Körperdaten, sie ergänzt die auf andere Art, vorzugsweise über eine Tastatur, eingegebenen Körperdaten des Benutzers wie beispielsweise Alter, Gewicht oder Geschlecht.

In den Monitor ist eine üblicherweise empirisch entwickelte Energieverbrauchs-Funktion einprogrammiert, wobei die variablen Grössen dieser Funktion sowohl einen oder mehrere Aktivitätsindikatoren als auch die Körperdaten und diesbezüglich insbesondere die Indikatorenreferenz beinhalten.

Ein vorteilhafter Ansatz für die Entwicklung einer Energieverbrauchs-Funktion basiert auf der Erkenntnis, dass für den aktivitätsabhängigen Energieumsatz der Aktivitätstyp von grosser Bedeutung ist. Aufgrund dieser Tatsache erlaubt eine zweiteilige Definition der Funktion ein systematisches Vorgehen bei deren Entwicklung: Zunächst werden verschiedene Aktivitätstypen unterschieden und es wird für jeden dieser Aktivitätstypen eine Formel zur Berechnung des Energieverbrauchs entwickelt. Diese Formelsammlung ist ein erster Teil der Energieverbrauchs-Funktion. Der zweite Teil der Funktion ist die Definition einer Prozedur zur Aktivitätsklassifizierung, die es erlaubt, die Aktivität des Benutzers anhand der Werte der Aktivitätsindikatoren und der Indikatorenreferenz einem der Aktivitätstypen zuzuordnen und die für diesen vorgesehene Berechnungsformel zu selektieren.

Die Klassifizierung der Aktivität des Benutzers zu einem bestimmten Zeitpunkt wird bevorzugt aufgrund der Werte der zu diesem Zeitpunkt gemessenen Aktivitätsindikatoren vorgenommen. Den vorgesehenen Aktivitätstypen sind dazu zusammenhängende Bereiche von Wertekombinationen der Aktivitätsindikatoren zugeordnet, die im Folgenden Indikatorenbereiche genannt werden. Die Grenzen dieser Bereiche werden anhand der Indikatorenreferenz personalisiert. Liegt die Wertekombination eines Indikatorentupels in einem so personalisierten Indikatorenbereich, so wird die Aktivität jenem Aktivitätstyp zugeordnet, für den dieser Indikatorenbereich definiert wurde.

Dieser Ansatz zur Zuordnung von Aktivitäten zu Aktivitätstypen liefert insbesondere dann gute Ergebnisse, wenn mindestens zwei verschiedenartige Aktivitätsindikatoren zur Verfügung stehen. Bevorzugt werden zumindest die Herzfrequenz und eine von der vertikalen Beschleunigung des Körpers abgeleitete Grösse als Aktivitätsindikatoren verwendet, da diese eine gute Unterscheidung von im Alltag relevanten und bei der Berechnung des Energieverbrauchs unterschiedlich zu behandelnden Aktivitätstypen ermöglichen. Beispielsweise lässt sich anhand dieser zwei Aktivitätsindikatoren gut erkennen, ob der Benutzer des Monitors bergab, auf der Ebene oder bergauf geht.

Überraschend hat sich gezeigt, dass die Hinzunahme der in Laufrichtung gemessenen, horizontalen Beschleunigung des Benutzers als dritten Aktivitätsindikator, in Ergänzung der beiden soeben genannten, nochmals eine wesentliche Verbesserung der Genauigkeit und Sicherheit bei der Zuordnung von Aktivitäten zu bestimmten Aktivitätstypen ermöglicht.

Die Indikatorenbereiche werden bevorzugt so definiert, dass sie unmittelbar aneinander grenzen und gemeinsam den Bereich der möglichen Wertekombinationen der Aktivitätsindikatoren abdecken, so dass jede Aktivität des Benutzers einem vorgesehenen Aktivitätstyp und somit einer Formel zur Ermittlung des Energieverbrauchs zugeordnet werden kann. Aufgrund ihrer Aktivitätsindikatoren a priori unbekannte, d.h. nicht vorgesehene Körperaktivitäten werden so dem aufgrund der Werte der Aktivitätsindikatoren nächstliegenden Aktivitätstyp zugeordnet. Bei Verwendung vorstehend genannten, bevorzugten Aktivitätsindikatoren (Herzfrequenz und vertiale Beschleunigung) lässt sich der bei Alltagstätigkeiten insgesamt anfallende Energieverbrauch damit sehr genau ermitteln.

Es ist aber auch möglich, die Indikatorenbereiche enger zu definieren, so dass gewisse Wertekombinationen der Aktivitätsindikatoren keinem bestimmten Aktivitätstyp zugeordnet werden können. Zur Schätzung des Energieverbrauchs kann für diese Fälle eine universell, d.h. unabhängig vom Aktivitätstyp einsetzbare Formel für die näherungsweise Berechnung des Energieverbrauchs vorgesehen sein.

Die Erfindung wird im Folgenden unter Bezugnahme auf die Figuren anhand eines Ausführungsbeispiels näher erläutert.
- Figur 1: zeigt ein Funktionsschema eines erfindungsgemässen Monitors mit Mitteln zur Messung der zwei Aktivitätsindikatoren Puls und Beschleunigung,
- Figur 2: zeigt in einem durch zwei Aktivitätsindikatoren aufgespannten Koordinatensystem die darin durch die Referenztupel bezeichneten Punkte und die personalisierten Grenzen der Indikatorenbereiche,
- Figur 3: zeigt in einem durch drei Aktivitätsindikatoren aufgespannten Koordinatensystem einen Eichpunkt und die personalisierten Grenzen von drei Indikatorenbereichen, und
- Figur 4: zeigt das Gehäuse des auf einem Brustgurt angebrachten Monitors und dessen Bedienfeld.

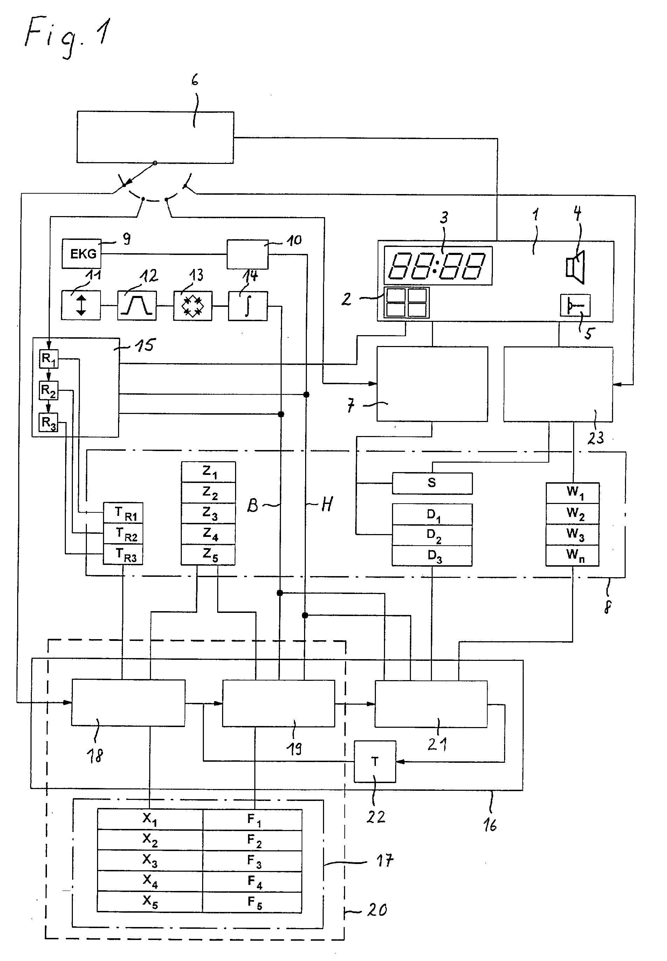

Das in Figur 1 dargestellte Funktionsdiagramm zeigt die funktionalen Elemente eines Körperaktivitätsmonitors und die Art und Weise wie diese miteinander in Verbindung stehen. Verbindungslinien ohne Pfeile stehen für Signal- bzw. Informationsflüsse, wogegen solche mit Pfeilen die chronologische Reihenfolge anzeigen, in der verschiedene Vorgänge ablaufen. Bevorzugt werden viele Elemente mittels Software realisiert, was bedeutet, dass der Monitor einen Microcontroller enthält, der jedoch nicht dargestellt ist.

Der Monitor verfügt über eine Ein/Ausgabe-Einheit 1 mit einer Tastatur 2 zur Bedienung des Monitors, einem Display 3, einem insbesondere bei der Eichung des Monitors nützlichen akkustischen Signalgeber 4 und einer Funk- oder Infrarot-Schnittstelle 5 für die Übertragung von Daten zwischen dem Monitor und anderen Geräten.

Eine Steuereinheit 6 ermöglicht über die Tastatur 2 die Wahl eines Betriebsmodus. Der Monitor hat vier Betriebsmodi: einen Programmiermodus, einen Eichungsmodus, einen Messmodus und einen Modus zur Datenanalyse.

Im Programmiermodus ermöglicht eine Programmiereinheit 7 über die Tastatur 2 die Eingabe von Daten, die dann in einem vorzugsweise nicht flüchtigen Datenspeicher 8, beispielsweise einem batterigepufferten RAM oder einem sogenannten Flash Memory, ablegt werden. Bevor der Monitor benutzt werden kann müssen insbesondere für die Berechnung des Energieverbrauchs benötigte, allgemeine Körperdaten D₁, D₂, D₃ des Benutzers wie beispielsweise sein Alter, sein Gewicht und sein Geschlecht eingegeben und gespeichert werden. Im vorliegenden Beispiel ist des Weiteren die Programmierung eines Werts für die vorgesehene Energieaufnahme S des Benutzers vorgesehen.

Ein erster Aktivitätsindikator für die Erfassung der Körperaktivität ist die Herzfrequenz, für deren Ermittlung eine geeignete Messeinrichtung vorgesehen ist. Im vorliegenden Beispiel besteht diese aus einer EKG-Einheit 9 und einer dieser nachgeschalteten Frequenzmess-Einheit 10. Als EKG-Einheit 9 sei hier allgemein eine Einheit zur Messung mindestens eines Herzstroms bezeichnet, mit der ein die Herzfrequenz enthaltendes Signal erzeugbar ist. Eine mögliche Alternative zur EKG-Einheit wäre ein Sensor zur Detektion von Druckwellen im Blutkreislauf, welche ebenfalls die Periodizität des Herzschlags aufweisen. In der Medizin spricht man in diesem Zusammenhang vom Puls. Für die Bestimmung des Energieverbrauchs ist die Unterscheidung von Puls und Herzfrequenz irrelevant, so dass die beiden Begriffe hier als Synonyme verwendet werden. Die Frequenzmess-Einheit 10 ist vorzugsweise so aufgebaut (oder als Teil der Software des Monitors implementiert), dass an ihrem Ausgang immer die aktuelle Herzfrequenz abgelesen werden kann. Beispielsweise könnte es sich um einen Zähler handeln, an dessen Ausgang immer die Zahl der in den letzten 60 Sekunden gezählten Herzschläge abgelesen werden kann. Um die Herzfrequenz schneller zu erfassen werden jedoch bevorzugt nicht während einer bestimmten Zeit die Herzschläge gezählt, sondern es wird der zeitliche Abstand von zwei Herzschlägen (oder der mittlere zeitliche Abstand von mindestens drei aufeinanderfolgenden Herzschlägen) gemessen und die Herzfrequenz durch Bilden des Kehrwerts ermittelt.

Als zweiter Aktivitätsindikator wird eine von der vertikalen Beschleunigung des Körpers abgeleitete Grösse verwendet, für deren Messung im Monitor ein in vertikaler Richtung sensibler Beschleunigungssensor 11 vorgesehen ist. Das Signal dieses Sensors 11 wird mit Vorteil zunächst durch ein Signalfilter 12 geschickt, dessen Frequenzgang so gestaltet ist, dass die für Körperaktivitäten bezeichnenden Beschleunigungsanteile betont und aktivitätsunabhängige Beschleunigungsanteile, insbesondere durch Umgebungseinflüsse hervorgerufene Körperbeschleunigungen, unterdrückt werden. Beispielsweise kann als Signalfilter 12 ein Bandpassfilter verwendet werden. Die untere Grenzfrequenz eines solchen Filters wird bevorzugt zwischen 0.5 und 1 Hz gewählt und die obere Grenzfrequenz zwischen 5 und 10 Hz.

Zur Bildung eines Aktivitätsindikators wird das so vorbehandelte Beschleunigungssignal anschliessend durch einen Gleichrichter 13 geschickt und das gleichgerichtete Beschleunigungssignal durch einen Integrator 14 über ein Zeitintervall aufintegriert. Die Länge dieses Zeitintervalls wird so gewählt, dass sich als Ergebnis der Integration ein für die Art der Bewegungsaktivität repräsentativer Wert ergibt. Bei einer langsamen, periodischen Körperaktivität (z.B. langsames Gehen) kann das Beschleunigungssignal eine Periodendauer von über 1 Sekunde haben und das Zeitintervall über das integriert wird, sollte ein Mehrfaches dieser Periodendauer betragen. Bevorzugt wird das gleichgerichtete Beschleunigungssignal über einen Zeitraum von mindestens 5 Sekunden integriert. Andererseits sollte das Integrationsintervall möglichst kurz sein, damit der Aktivitätsindikator für die Art der Körperaktivität zu einem bestimmten Zeitpunkt repräsentativ ist und Wechsel der Aktivitätsart während eines Integrationsintervalls die Ausnahme bleiben. Es ist daher von Vorteil, wenn das Integrationsintervall nicht länger als 15 Sekunden ist. Bevorzugt wird das gleichgerichtete Beschleunigungssignal durch den Integrator 14 über einen Zeitraum von 8 bis 12 Sekunden integriert.

Diese Bestimmung des von der vertikalen Beschleunigung abhängigen Aktivitätsindikators durch den Integrator 14 wird zur kontinuierlichen Erfassung der Körperaktivität periodisch wiederholt, was nachstehend noch näher erläutert wird. An Stelle des in vertikaler Richtung sensiblen Beschleunigungssensors 11, oder zusätzlich zu diesem, könnte auch ein in Laufrichtung, d.h. in Bezug auf horizontale vorwärts/rückwärts Bewegungen sensibler Beschleunigungssensor eingesetzt und so auf die gleiche Weise ein von der Beschleunigung in Laufrichtung abhängiger Aktivitätsindikator ermittelt werden.

Der Übergang von analoger Messtechnik auf digitale Signalverarbeitung kann an verschiedenen Stellen geschehen. Bevorzugt wird so viel wie möglich durch einen programmierbaren Microcontroller durchgeführt, da so Anpassungen einfach durch die Software vorgenommen werden können und der Aufwand an analoger Schaltungstechnik reduziert werden kann. So wird das Signalfilter 12 bevorzugt als digitales Filter (beispielsweise ein IIR-Filter) implementiert. Auch der Integrator 14 wird am einfachsten mittels Software realisiert: Er tastet das gefilterte und gleichgerichtete Beschleunigungssignal periodisch ab und summiert die Abtastwerte während des Integrationsintervalls laufend auf. Die Abtastrate des Integrators 14 muss deutlich über der höchsten Frequenzkomponente des zu integrierenden Signals liegen, sie beträgt vorzugsweise mindestens das Doppelte der oberen Grenzfrequenz des Bandpassfilters 12 (also eine Samplingrate von mindestens 10 Hz bei einem Bandpassfilter 12 mit einer oberen Grenzfrequenz von 5 Hz).

Zusätzlich zur bereits beschriebenen Programmierung muss der Monitor vor dem ersten Einsatz auch auf den Benutzer geeicht werden. Bei dieser Eichung wird eine hier als Indikatorenreferenz bezeichnete Information zur benutzerspezifischen Sensibilität der Aktivitätsindikatoren ermittelt und gespeichert. Es handelt sich um eine Information zur körperlichen Konstitution des Benutzers des Monitors und somit um einen Teil der Körperdaten. Wird unter der Programmierung des Monitors allgemein die Eingabe von zur Berechnung des Energieverbrauchs benötigten Körperdaten des Benutzers verstanden, so ist die Eichung des Monitors ein Teil des Programmiervorgangs. Im Gegensatz zu den über die Tastatur 2 programmierten Körperdaten (Alter, Gewicht, etc.), die dem Benutzer bekannt sind, muss die Indikatorenreferenz jedoch von den bei bestimmten Referenzaktivitäten des Benutzers messbaren Aktivitätsindikatoren abgeleitet werden.

Als Referenzaktivitäten werden bevorzugt einer oder mehrere Intensitätsgrade der Fortbewegung zu Fuss vorgesehen. Die Fortbewegung zu Fuss ist im Alltag die häufigste körperliche Betätigung und die sich dabei einstellenden persönlichen Referenzwerte der Aktivitätsindikatoren sind für eine genaue Berechnung des längerfristigen (und somit primär von der Alltagsaktivität abhängigen) Energieverbrauchs daher besonders wertvoll. Im Alltag ist das Gehen die weitaus häufigste Form der Fortbewegung zu Fuss, andere Formen wie beispielsweise das Joggen oder Laufen sind auf in der Regel kürzere Phasen sportlicher Aktivität beschränkt. Bevorzugt wird daher das Gehen als Referenzaktivität gewählt, oder verschiedene Intensitätsgrade des Gehens wenn mehrere Referenzaktivitäten vorgesehen sind.

Im Eichungsmodus übt der Benutzer des Monitors Während drei Eichungszyklen je eine Referenzaktivität R₁, R₂, R₃ aus. Der Ablauf dieser Eichungszyklen wird durch eine Eichungseinheit 15 gesteuert. Der erste Eichungszyklus wird vom Benutzer durch das Drücken einer Taste auf der Tastatur 2 gestartet, mit dem er dem Monitor den Beginn der ersten Referenzaktivität signalisiert. Nach einer Aktivitätsperiode, die ausreichend lang ist um sicherzustellen, dass die Aktivitätsindikatoren die der Referenzaktivität entsprechenden Werte angenommen haben (was beispielsweise für die Herzfrequenz einige Minuten dauern kann), werden durch die Eichungseinheit die am Ausgang des Frequenzmess-Einheit 10 bzw. des Integrators 14 verfügbaren, aktuellen Aktivitätsindikatoren als für die erste Referenzaktivität R₁ ermitteltes Referenztupel T_{R1} gespeichert. Gleichzeitig sendet die Eichungseinheit über den Signalgeber 4 ein akkustisches Signal aus, das dem Benutzer das Ende der ersten Referenzmessung mitteilt.

Darauf folgen in gleicher Weise der zweite und der dritte Eichungszyklus, bei denen die zu den Referenzaktivitäten R₂ und R₃ ermittelten Referenztupel T_{R2} und T_{R3} gespeichert werden. Insbesondere wenn einer der Aktivitätsindikatoren die Herzfrequenz ist, werden die Referenzaktivitäten bevorzugt in der Reihenfolge ihrer Intensität ausgeübt, wobei mit der Referenzaktivität mit der geringsten Intensität (z.B. langsames Gehen) begonnen wird. Werden die verschiedenen Referenzaktivitäten unmittelbar nacheinander ausgeübt, so kann damit die Zeit, während der diese Referenzaktivitäten vom Benutzer ausgeübt werden müssen, minimiert werden. Es ist daher von Vorteil, wenn das genannte akkustische Signal dem Benutzer nicht nur das Ende der ersten Referenzmessung anzeigt sondern zugleich als Anweisung zum Beginn mit der zweiten Referenzaktivität zu verstehen ist. Dasselbe gilt auch für die nachfolgenden Wechsel der Referenzaktivität, bis alle Referenzmessungen abgeschlossen sind. Der Benutzer signalisiert dem Monitor also lediglich den Beginn seiner ersten Referenzaktivität und wechselt danach bei jedem akkustischen Signal zur nächst intensiveren Referenzaktivität. Das Ende der letzten Referenzmessung kann dem Benutzer durch ein anderes akkustisches Signal mitgeteilt werden.

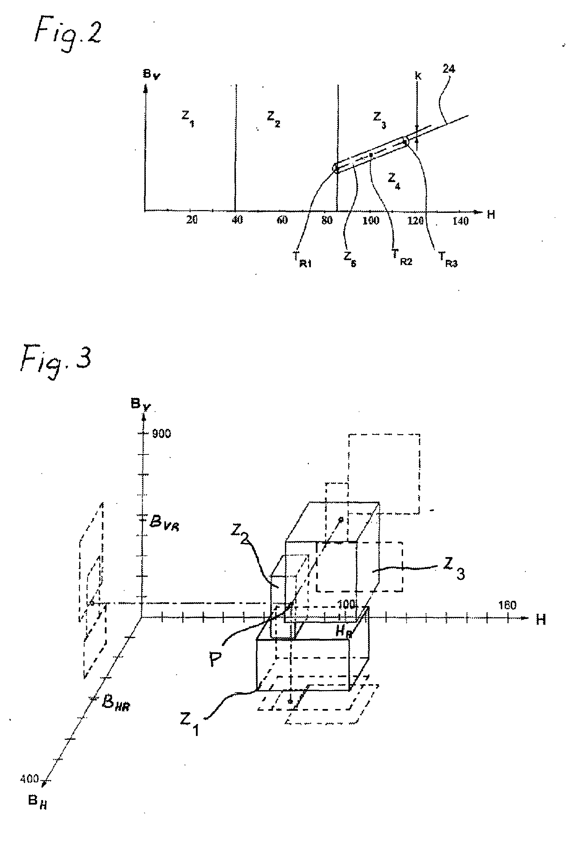

In diesem Beispiel werden direkt die für die Referenzaktivitäten ermittelten Werte der Aktivitätsindikatoren als Indikatorenreferenz gespeichert. Alternativ dazu wäre es jedoch auch möglich, von diesen Referenztupeln T_{R1}, T_{R2}, T_{R3} eine andere Indikatorenreferenz abzuleiten und im Speicher abzulegen, was nachstehend zu Figur 2 noch näher erläutert wird.

Nach erfolgter Eichung ist der Monitor einsatzbereit. Im Messmodus wird durch eine Verbrauchsermittlungseinheit 16 periodisch der in der letzten Messperiode T angefallene Energieverbrauch W berechnet und das Ergebnis im Speicher 8 abgelegt. Für diese Berechnung hat die Verbrauchsermittlungseinheit 16 Zugriff auf einen Speicher 17 (bei dem es sich um ein ROM handeln kann, insbesondere um dasselbe ROM, in dem sich auch der gesamte Programmcode des Microcontrollers befindet), in dem für fünf vorgesehene Aktivitätstypen Formeln F₁ - F₅ zur Berechnung des Energieverbrauchs sowie für die Zuordnung der Aktivitäten des Benutzers zu diesen Aktivitätstypen festgelegte Indikatorenbereiche X₁ - X₅ definiert sind.

Die Formeln F₁ - F₅ enthalten Variablen, bei denen es sich, je nach Aktivitätstyp für den eine Formel vorgesehen ist, um einen oder mehrere der Aktivitätsindikatoren und/oder eine oder mehrere Grössen aus den gespeicherten Körperdaten wie Alter, Geschlecht und Gewicht handeln kann. Einen möglichen Ansatz für die Entwicklung dieser Formeln bietet die Regressionsanalyse. Dazu werden bei einer Gruppe von Testpersonen für verschiedene Intensitätsgrade eines Aktivitätstyps (für den eine Berechnungsformel gesucht wird) die Aktivitätsindikatoren gemessen und zudem wird der mit der betreffenden Aktivität verbundene Energieverbrauch gemessen, beispielsweise mittels indirekter Kalorimetrie (O₂ und CO₂ Messung). Anhand des nunmehr bekannten Energieverbrauchs und der zugehörigen Werte der in der Formel zu berücksichtigenden Grössen können durch multifaktorielle Regression die Faktoren ermittelt werden, mit denen diese Grössen zum Energieverbrauch beitragen. Wird in einer Formel das Geschlecht berücksichtigt, so kann dieser Grösse bei einer weiblichen Person der Wert 1 und bei einer männlichen Person der Wert 0 gegeben werden. In erster Näherung kann ein lineares Regressionsmodell verwendet werden. Dieses Vorgehen wird für jeden der vom Monitor unterschiedenen Aktivitätstypen wiederholt, in diesem Beispiel fünf mal, zur Entwicklung der Formeln F₁ - F₅.

Die Berechnung läuft in drei Schritten ab, für die in der Verbrauchsermittlungseinheit 16 drei funktionale Untereinheiten vorgesehen sind: eine Personalisierungseinheit 18, eine Klassifizierungseinheit 19 und eine Auswertungseinheit 21.

Zunächst werden durch die Personalisierungseinheit 18 anhand der Indikatorenreferenz die Grenzen der Indikatorenbereiche X₁ - X₅ der körperlichen Konstitution des Benutzers angepasst. Dieser Schritt wird zu Figur 2 noch näher erläutert. Die Definitionen der so personalisierten Indikatorenbereiche Z₁ - Z₅ werden im Speicher 8 gespeichert. Die Personalisierung der Indikatorenbereiche muss nach der Eichung des Monitors lediglich einmal durchgeführt werden. Dieser Schritt könnte daher auch durch die Eichungseinheit, im Anschluss an die Messung der Referenztupel durchgeführt werden, so dass auf eine dauerhafte Speicherung der Referenztupel T_{R1}, T_{R2}, T_{R3} verzichtet werden könnte. Die Indikatorenreferenz wäre dann direkt als Teil der personalisierten Indikatorenbereiche Z₁ - Z₅ gespeichert.

Die Klassifizierungseinheit 19 vergleicht die an den Ausgängen der Frequenzmess-Einheit 10 bzw. des Integrators 14 ablesbaren, aktuellen Aktivitätsindikatoren mit den personalisierten Indikatorenbereichen Z₁ - Z₅ und identifiziert so den durch das Indikatorentupel indizierten Indikatorenbereich. Damit wird die aktuelle Aktivität des Benutzers jenem Aktivitätstyp zugeordnet, für den der betreffende Indikatorenbereich definiert ist. Die für diesen Aktivitätstyp vorgesehene Berechnungsformel wird dann von der Klassifizierungseinheit 19 an die Auswertungseinheit 21 übergeben. Die in dem mit gestrichelter Linie gezeichneten Feld 20 enthaltenen Elemente, also die Personalisierungseinheit 18 und die Klassifizierungseinheit 19, bei denen es sich im Grunde um Prozeduren handelt, sowie die Berechnungsformeln F₁ - F₅ und die zugehörigen Indikatorenbereiche X₁ - X₅, bilden zusammen die Energieverbrauchsfunktion des Monitors.

Die Auswertungseinheit 21 wertet die ihr übergebene Berechnungsformel numerisch aus und legt das Ergebnis, einen Wert für den seit der letzten Energieverbrauchsmessung angefallenen Energieverbrauch W, im Speicher 8 ab.

Um kontinuierlich den Energieverbrauch zu ermitteln, werden die letzten zwei Schritte, also die Aktivitätsklassifizierung und Formelauswertung, periodisch wiederholt. Die Zeit T zwischen zwei solchen Messzyklen wird durch einen Timer 22 so kurz eingestellt, dass auch kurzzeitige Körperaktivitäten erkannt werden. Bevorzugt beträgt die Zeit zwischen zwei Messzyklen nicht mehr als 15 Sekunden. Für jeden dieser Zyklen wird der von der vertikalen Bewegungsaktivität abhängige Aktivitätsindikator B_{V} neu ermittelt, was bedeutet, dass auch der Integrator 14 durch denselben Timer 22 jeweils nach der Zeit T neu gestartet wird. Bei dem Integrator kann es sich um eine Programmroutine handeln, die periodisch die Werte des gleichgerichteten Beschleunigungssignals abtastet, die Abtastwerte laufend addiert und nach N Abtastzyklen die Summe ausgibt und einen Ermittlungszyklus der Verbrauchsermittlungseinheit 16 auslöst um aufgrund dieser als Aktivitätsindikator dienenden Summe (und gegebenenfalls weiterer Aktivitätsindikatoren, in diesem Beispiel die Herzfrequenz) den aktuellen Energieverbrauch zu berechnen.

Zur lückenlosen Überwachung der Körperaktivität wird die Zeit T zwischen zwei Messzyklen bevorzugt gleich der Länge eines Integrationsintervalls des Integrators 14 gewählt. Das gleichgerichtete Beschleunigungssignal wird durch den Integrator 14 dabei kontinuierlich abgetastet und die Abtastwerte werden kontinuierlich aufaddiert, wobei jeweils nach N Abtastwerten die Summe als neuer Wert des Aktivitätsindikators B_{V} ausgegeben, ein Ermittlungszyklus der Verbrauchsermittlungseinheit 16 ausgelöst und der Integrator 14 wieder zurückgesetzt wird. In diesem Fall wird kein separater Timer 22 benötigt, da sich die Zeit T bei einer gegebenen Samplingfrequenz fs des Integrators 14 durch geeignete Wahl der Anzahl in einem Integrationsintervall enthaltener Samples N festlegen lässt: T = N / fₛ.

Bei kontinuierlicher Abtastung des Beschleunigungssignals durch den Integrator 14 könnte dieser auch als FIR-Filter mit N Filterkoeffizienten realisiert werden, wobei aus dem Ausgangsdatenstrom dieses FIR-Filters für die soeben beschriebene Variante (wo T = N / fₛ) lediglich einer von N Werten als Aktivitätsindikator verwendet würde. Der Rechenaufwand für eine Implementation als FIR-Filter würde sich in diesem Fall also nicht lohnen. Die Verwendung eines FIR-Filters als Integrator 14 würde es jedoch ermöglichen, die Zeit T zwischen zwei Messzyklen kürzer als das Integrationsintervall zu wählen (T < N / fₛ).

In der Regel trägt der Benutzer den Monitor während einer längeren Messperiode, beispielsweise von Morgens bis Abends. Nach einer solchen Messperiode kann der Benutzer den für die Datenanalyse vorgesehenen Betriebsmodus des Monitors wählen. Eine Analyseeinheit 23 ermöglicht über die Tastatur 2 das Abrufen verschiedener Informationen zum Energieverbrauch des Benutzers, die dann auf dem Display 2 angezeigt werden. Da die Verbrauchsermittlungseinheit 16 die während der Überwachung periodisch ermittelten Werte P₁ - Pₙ des Energieverbrauchs einzeln im Speicher 8 abgelegt hat, kann durch die Analyseeinheit 23 nicht nur der kumulierte Energieverbrauch berechnet, sondern auch dessen Entstehung analysiert und angezeigt oder über die Schnittstelle 5 zur weiteren Bearbeitung auf einen Personalcomputer übertragen werden. Weitere Einzelheiten zur Auswertung und Anzeige von Daten sind nachstehend zu Figur 3 beschrieben.

Zu Figur 1 ist schliesslich zu betonen, dass die verschiedenen funktionalen Einheiten wie Programmiereinheit 7, Eichungseinheit 15, Verbrauchsermittlungseinheit 16, etc. vorzugsweise Elemente eines einzigen Computerprogramms sind. Die verschiedenen Abläufe in diesem Programm sind im Interesse einer möglichst einfachen Darstellung der Zusammenhänge als Funktionen verschiedener Einheiten dargestellt, dieselben Abläufe könnten jedoch auch anders gruppiert und bezeichnet werden, ohne dass sich inhaltlich etwas ändert. Wesentlich sind die anhand der Figur erklärten Abläufe bei der Datenverarbeitung und nicht deren Zuordnung zu funktionalen Einheiten.

In Figur 2 ist das durch die beiden Aktivitätsindikatoren des in Figur 1 beschriebenen Monitors aufgespannte Koordinatensystem grafisch dargestellt, wobei die Herzfrequenz H auf der horizontalen und der von der vertikalen Beschleunigung abgeleitete Aktivitätsindikator B_{V} auf der vertikalen Achse aufgetragen sind. Die Indikatorentupel bezeichnen Punkte in diesem Koordinatensystem. Eingezeichnet sind die drei durch die Referenztupel T_{R1}, T_{R2}, T_{R3} bezeichneten Referenzpunkte. Bei den Referenzaktivitäten R₁, R₂, R₃, für die diese Referenztupel gemessen wurden, handelt es sich um verschiedene Intensitätsgrade des Gehens (langsames, schnelleres und schnelles Gehen mit 2, 4 und 6 km pro Stunde).

Es fällt auf, dass die verschiedenen Intensitätsgraden des Gehens entsprechenden Indikatorentupel Punkte bezeichnen, die im Wesentlichen auf einer Geraden 24 liegen. Die Lage des von einem Indikatorentupels bezeichneten Punkts in Bezug auf diese Gerade sagt etwas über die Art der Körperaktivität des Benutzers aus: Liegt die Herzfrequenz unter dem bei langsamem Gehen gemessenen Wert, so ist daraus zu schliessen, dass sich der Benutzer ruhig verhält. Ist die Herzfrequenz höher, so ist eine stärkere körperliche Aktivität zu vermuten, wobei, ausgehend von der Annahme, dass die Hauptaktivität des Benutzers das Gehen ist, zunächst drei Fälle unterschieden werden können: Liegt der Punkt auf der Geraden 24, zwischen den für langsames und schnelles Gehen ermittelten Referenzpunkten T_{R1} und T_{R3}, so ist der Benutzer vermutlich am Gehen. Liegt der Punkt über oder unter der Geraden 24 so geht der Benutzer vermutlich bergab bzw. bergauf (im Vergleich zur Geraden 24 niedrigere bzw. höhere Herzfrequenz H bei gleicher Bewegungsintensität B).

So lassen sich zur Klassifizierung der Aktivitäten des Benutzers zusammenhängende Bereiche Z1 - Z5 der Wertekombinationen von Herzfrequenz H und Bewegungsintensität B_{V} definieren, wobei die Grenzen dieser Indikatorenbereiche zumindest teilweise anhand der Referenztupel definiert sind.

Die Gerade 24 ist für die Definition der Indikatorenbereiche besonders wertvoll. Bei der Eichung des Monitors wird daher eine Indikatorenreferenz gespeichert, welche die Definition dieser Geraden 24 oder, allgemeiner formuliert, die Definition einer durch die von den Referenztupeln bezeichneten Punkte gelegten Eichkurve 24 beinhaltet.

Die Punkte werden selten genau auf einer Geraden liegen, die tatsächliche Beziehung von Herzfrequenz und Bewegungsintensität lässt sich jedoch näherungsweise durch eine Gerade ersetzen, so dass sich der für die Aktivitätsklassifizierung nötige Rechenaufwand im Rahmen hält. Bei zwei Referenzaktivitäten ist diese Gerade durch die beiden Referenztupel definiert und wenn, wie im vorliegenden Beispiel, mehr als zwei Intensitätsgrade des Gehens als Referenzaktivitäten vorgesehen sind, so kann eine durch die Punkte der Referenztupel gelegte Regressionsgerade verwendet werden.

Eine Eichkurve ist insbesondere dann eine sinnvolle Referenz, wenn sie von Referenztupeln abgeleitet ist, die bei Referenzaktivitäten desselben Typs, im vorliegenden Fall verschiedenen Intensitätsgraden des Gehens, gemessen wurden. Die Eichkurve beschreibt dann für diesen Aktivitätstyp die gegenseitige Abhängigkeit der Aktivitätsindikatoren. Im Beispiel ist das die Funktion B_{R}(H), die für den Aktivitätstyp R den Wert der Bewegungsintensität B_{V} in Funktion der Herzfrequenz H definiert.

Für die bereits erwähnten Aktivitätstypen können so die folgenden Indikatorenbereiche Z₁ - Z₅ definiert werden:

| Aktivität | Indikatorenbereich | |
|---|---|---|
| Schlaf | X₁: | Kein Puls gemessen |
| Ruhe | X₂: | 40 Hz < H < H_{R1} |
| Bergab gehen | X₃: | H_{R1} < H ; B_{V} > B_{R} (H) |
| Bergauf gehen | X₄: | H_{R1} < H ; B_{V} < B_{R} (H) |
| Gehen in der Ebene | X₅: | H_{R1} < H < H_{R3} ; B_{V} = B_{R}(H) +/- k |

Ist kein Puls messbar, so ist zu vermuten, dass der Benutzer den Monitor abgelegt hat. Wird der Monitor nur zum Schlafen abgelegt, so kann für den Indikatorenbereich X₁ eine zur Berechnung des Energieverbrauchs beim Schlafen vorgesehene Berechnungsformel vorgesehen werden. Damit ist es möglich, während mehreren Tagen und selbst Wochen kontinuierlich den Energieverbrauch des Benutzers zu messen, unter der Voraussetzung, dass der Monitor lediglich während der Schlafperioden abgelegt wird.

Die Indikatorenbereiche sind in dieser Tabelle mittels Variablen für die Werte der Referenztupel und somit in allgemeiner Form definiert. Sie sind daher nicht wie in der Figur mit Z₁ - Z₅, sondern mit X₁ - X₅ bezeichnet. In dieser Form könnten die Indikatorenbereiche beispielsweise in dem zu Figur 1 erwähnten Speicher 17 des Monitors definiert sein. Die als Personalisierung bezeichnete Anpassung dieser Bereiche an den Benutzer des Monitors erfolgt durch das Einsetzen von konkreten, aus der Indikatorenreferenz bekannten Werten für die Variablen.

Wie vorstehend erwähnt, wird für die Referenzaktivitäten eine im Alltag dominierende Aktivitätsart bevorzugt und aus demselben Grund wird für dieselbe Aktivitätsart mit Vorteil auch eine spezielle Formel zur Berechnung des dabei anfallenden Energieverbrauchs entwickelt. Der für die Zuordnung von Aktivitäten zur Referenzaktivitätsart vorgesehene Indikatorenbereich wird dabei bevorzugt durch einen Toleranzwert k für die maximale Abweichung von der Eichkurve 24 definiert.

Eine Alternative zur Definition der Indikatorenbereiche X₁ - X₅ durch abstrakte Regeln bzw. Formeln gemäss vorstehender Tabelle besteht darin, in Bezug auf eine Standard-Eichkurve eines durchschnittlichen Benutzers konkrete, d.h. durch Zahlenwerte bestimmte Standard-Indikatorenbereiche X₁ - X₅ festzulegen. Durch eine Transformation der Bereichsgrenzen werden davon dann personalisierte Indikatorenbereiche Z₁ - Z₅ abgeleitet, deren Lage in Bezug auf die Eichkurve des Benutzers der Lage der entsprechenden Standard-Indikatorenbereiche in Bezug auf die Standard-Eichkurve entspricht.

Zu Figur 2 ist schliesslich zu erwähnen, dass die genannten Aktivitätstypen auch anders definiert werden könnten, womit sich ein anderer, möglicherweise komplexerer Verlauf der Grenzen der Indikatorenbereiche ergeben kann. Insbesondere kann sich bei Berücksichtigung einer grösseren Anzahl vom Monitor zu unterscheidender Aktivitätstypen auch eine andere Definition der Indikatorenbereiche ergeben. Die beschriebene Methode zur Aktivitätsklassifizierung ist in analoger Weise auch anwendbar, wenn an Stelle des von der vertikalen Körperbeschleunigung abhängigen Aktivitätsindikators B_{V} ein von der Körperbeschleunigung in Laufrichtung abhängiger Aktivitätsindikator (oder ein anderer Aktivitätsindikator) verwendet wird. Letzterer könnte, bei entsprechender Anpassung der Definitionen der Indikatorenbereiche, auch an Stelle der Herzfrequenz verwendet werden. Alle drei genannten Grössen (Herzfrequenz und Beschleunigung, vertikal und in Laufrichtung) können als Aktivitätsindikatoren verwendet werden, wobei zwei davon auf die beschriebene Art zur Aktivitätsklassifikation verwendet werden und der dritte (und möglicherweise noch weitere) lediglich über in den Formeln F₁ - F₅ enthaltene Variablen in die Berechnung des Energieverbrauchs mit einfliesst.

In einer weiter verbesserten Ausführungsform des Monitors wird zusätzlich zu den beiden im vorstehend beschriebenen Beispiel verwendeten Aktivitätsindikatoren Herzfrequenz und vertikale Beschleunigung auch die in Laufrichtung gemessene, horizontale Körperbeschleunigung als dritter Aktivitätsindikator nicht nur in den Formeln F₁ - F₅ zur Berechnung des Energieverbrauchs sondern auch bei der Aktivitätsklassifizierung mit berücksichtigt. Das Funktionsdiagramm dieses Monitors entspricht dem in Figur 1 gezeigten, wobei neben dem Beschleunigungssensor 11 zusätzlich ein (nicht dargestellter) Beschleunigungssensor für die Messung der horizontalen Körperbeschleunigung vorgesehen ist, der in gleicher Weise über ein Signalfilter, einen Gleichrichter und einen Integrator einen von der horizontalen Beschleunigung abgeleiteten Aktivitätsindikator bereitstellt.

Bei der Eichung des Monitors werden, wie zu Fig. 1 beschrieben, für drei Referenzaktivitäten R₁, R₂, R₃ die zugehörigen Aktivitätsindikatoren ermittelt. Die erste Referenzaktivität R₁ ist langsames Gehen (Schlendern, langsames Spazieren). Die zweite Referenzaktivität R₂ ist moderates Gehen, was dem zielorientierten Gehen, z.B. von einem Ort an den andern, entspricht. Die dritte Referenzaktivität R₃ ist schnelles Gehen, wozu der Benutzer aufgefordert wird, in Eile zu gehen. Jede dieser Referenzaktivitäten wird für etwa 3 Minuten ausgeübt und aus den während der letzten Minute gemessenen Werten der Aktivitätsindikatoren werden die jeweiligen Durchschnitte berechnet und als Referenztupel T_{R1}, T_{R2}, T_{R3} gespeichert.

Im Messmodus wird auch hier durch die Verbrauchsermittlungseinheit 16 periodisch der Energieverbrauch W ermittelt. Der Speicher 17 enthält (in Abweichung von Fig. 1) für drei vorgesehene Aktivitätstypen Formeln F₁ - F₃ zur Berechnung des Energieverbrauchs und für die Zuordnung der Aktivitäten des Benutzers zu diesen Aktivitätstypen festgelegte Indikatorenbereiche X₁ - X₃. Zusätzlich enthält der Speicher 17 eine Universalformel für die Berechnung des Energieverbrauchs (in Fig. 1 nicht dargestellt), die verwendet wird, falls sich die Aktivität des Benutzers aufgrund der gemessenen Aktivitätsindikatoren keinem der vorgesehenen Aktivitätstypen zuordnen lässt. Diese Universalformel kann, analog dem zu Fig. 1 für die Formeln zu bestimmten Aktivitätstypen gesagten, ebenfalls mittels Regressionsanalyse entwickelt werden, wobei für diese Analyse vorzugsweise auf die Gesamtheit der für die Entwicklung der aktivitätsspezifischen Formeln F1 - F3 verwendeten Messdaten abgestellt wird.

Die Berechnung des Energieverbrauchs verläuft wie zu Fig. 1 beschrieben, wobei die Personalisierung der Indikatorenbereiche X₁ - X₃ durch die Personalisierungseinheit 18 und die anschliessende Klassifizierung der Aktivität des Benutzers durch die Klassifizierungseinheit 19 im Folgenden unter Bezugnahme auf Figur 3 näher erläutert werden.

Figur 3 zeigt das durch die drei Aktivitätsindikatoren Herzfrequenz H, vertikale Beschleunigung B_{V} und horizontale Beschleunigung B_{H} aufgespannte Koordinatensystem, wobei die Herzfrequenz H auf der horizontalen Achse, der von der vertikalen Beschleunigung abgeleitete Aktivitätsindikator B_{V} auf der vertikalen Achse und der von der horizontalen Beschleunigung abgeleitete Aktivitätsindikator B_{H} auf der dritten Achse aufgetragen sind. Die Indikatorentupel bezeichnen Punkte in diesem Koordinatensystem. Ein im Folgenden als Eichpunkt P bezeichneter Referenzpunkt in diesem Koordinatensystem ist durch die arithmetischen Mittelwerte H_{R}, B_{VR}, B_{HR} der für die Referenzaktivitäten R₁, R₂, R₃ anlässlich der Eichung des Monitors gemessenen Werte der Aktivitätsindikatoren definiert.

Die in den Grössen der drei Aktivitätsindikatoren gemessenen Ausdehnungen der personalisierten Indikatorenbereiche sind für alle Benutzer gleich, sie werden bei der Personalisierung der im Speicher 17 (Fig. 1) gespeicherten Indikatorenbereiche X₁ - X₃ nicht angepasst. Dasselbe gilt für die auf den Eichpunkt bezogene Lage der Indikatorenbereiche in dem Koordinatensystem. Jeder der Indikatorenbereiche X₁ - X₃ ist durch in Bezug auf den Eichpunkt angegebene Werteintervalle der Aktivitätsindikatoren definiert und im Speicher 17 in Tabellenform abgelegt:

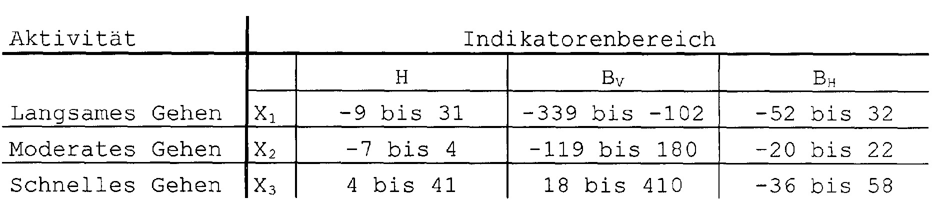

Die Herzfrequenz ist in Hertz angegeben und die Werte der beiden anderen Aktivitätsindikatoren sind von der jeweiligen Beschleunigung abgeleitete, für die Bewegungsintensität repräsentative Werte, deren Messung zu Figur 1 näher erläutert wurde. Diese Indikatorenbereiche wurden empirisch bestimmt, wobei darauf geachtet wurde, dass sich die Bereiche nicht überschneiden.

Zur Personalisierung der Indikatorenbereiche X₁ - X₃ wird durch die Personalisierungseinheit 18 zunächst anhand der als Indikatorenreferenz gespeicherten Referenztupel T_{R1}, T_{R2}, T_{R3} der genannte Eichpunkt P berechnet. Der in Figur 3 eingezeichnete Eichpunkt liegt bei H_{R} = 97 Hz, B_{VR} = 488 und B_{HR} = 202. Durch Verschieben der die Indikatorenbereiche X₁ - X₃ definierenden Intervalle um diese Werte werden danach die Grenzen der personalisierten Intervalle ermittelt, welche die personalisierten Indikatorenbereiche Z₁ - Z₃ definieren.

Nach der Personalisierung der Indikatorenbereiche wird durch die Klassifizierungseinheit 19 periodisch der durch die aktuellen Werte der Aktivitätsindikatoren indizierte, personalisierte Indikatorenbereich Z₁ - Z₃ bestimmt und die diesem zugeordnete Formel F₁ - F₃ für die Berechnung des Energieverbrauchs selektiert. Ein Indikatorenbereich Z₁ - Z₃ ist eindeutig indiziert, wenn die Werte von allen drei Aktivitätsindikatoren in den entsprechenden, den personalisierten Indikatorenbereich definierenden Werteintervallen liegen. Liegt der durch ein Indikatorentupel definierte Punkt des Koordinatensystems in keinem der (personalisierten) Indikatorenbereiche, so kann die für diesen Fall vorgesehene Universalformel zur näherungsweisen Berechnung des Energieverbrauchs herangezogen werden.

Als Alternative dazu kann bei mangelnder Eindeutigkeit geprüft werden, ob im vorhergehenden Ermittlungszyklus eine eindeutige Zuordnung möglich war. Ist dies der Fall, so wird an Stelle der Universalformel erneut die für diesen Aktivitätstyp vorgesehene Formel selektiert, sofern erneut zumindest zwei der Aktivitätsindikatoren in den diesen Indikatorenbereich definierenden, personalisierten Werteintervallen liegen. Dieses Verfahren zur Fehlerkorrektur kann auch auf den zweiten und weitere auf einen Ermittlungszyklus mit eindeutiger Zuordnung folgende Ermittlungszyklen ausgedehnt werden.

In Ergänzung der in Figur 3 dargestellten Indikatorenbereiche Z₁ - Z₃ könnten für weitere Aktivitätstypen zusätzliche Indikatorenbereiche und zugehörige Formeln zur Berechnung des Energieverbrauchs vorgesehen sein. Die Indikatorenbereiche Z1 - Z3 sind in diesem Beispiel durch Werteintervalle der Aktivitätsindikatoren definiert, was den Vorteil hat, dass sich die Indizierung eines solchen Bereichs durch ein Indikatorentupel mittels einfacher Vergleichsoperationen überprüfen lässt. Komplexere Definitionen von einem oder mehreren Indikatorenbereichen sind jedoch ebenfalls möglich, wobei deren Personalisierung in analoger Weise durch Verschieben des Nullpunkts zur Position des Eichpunkts erfolgen kann.

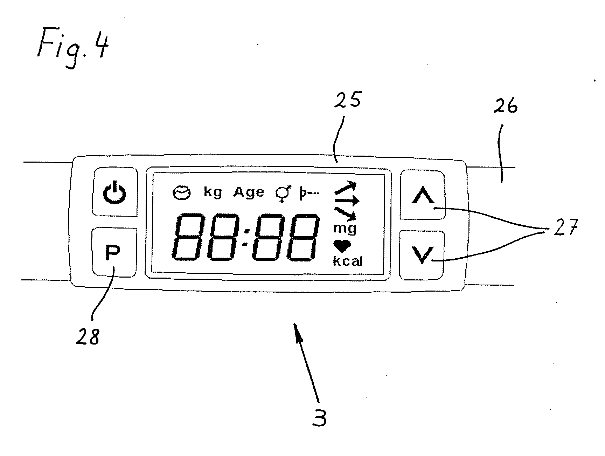

Figur 4 zeigt eine mögliche Bauform eines Monitors mit der zu Figur 1 erläuterten Funktionalität. Der Monitor hat ein Gehäuse 25, das auf einem Brustgurt 26 montiert ist. Dieser wird vom Benutzer unter den Kleidern, direkt auf der Haut getragen. Der in vertikaler Richtung sensible Beschleunigungssensor 11 ist in dem Gehäuse 25 angeordnet und auf dem Brustgurt sind die nicht dargestellten Elektroden der EKG-Einheit 9 vorgesehen. Weitere Sensoren zur Ermittlung anderer Aktivitätsindikatoren, insbesondere ein in Laufrichtung sensibler Beschleunigungssensor, können ebenfalls im Gehäuse 25 untergebracht sein. Zur Bedienung der Funktionen dieses Monitors sind mehrere Tasten 2 vorgesehen. Eine dieser Tasten 28 ist zur Wahl des Betriebsmodus des Monitors vorgesehen und mit den beiden Pfeiltasten 27 können verschiedene auf dem Display 3 angezeigte Funktionen des Monitors ausgewählt oder bei der Programmierung die Werte von Körperdaten wie Alter oder Körpergewicht eingestellt werden.

Zu Figur 1 wurden der Messmodus und der Modus zur Datenanalyse als separate Betriebsmodi beschrieben. Die Datenanalyse und insbesondere die fortlaufende Anzeige des kumulierten Energieverbrauchs kann jedoch auch während einer Messphase erfolgen, so dass der Benutzer jederzeit Informationen zum aktuellen Stand seines Energieverbrauchs oder seiner Energiebilanz zur Verfügung hat.

Für die Berechnung der Energiebilanz benötigt die Analyseeinheit 23 (Fig. 1) zusätzlich zu den periodisch gemessenen Energieverbrauchswerten W auch eine Information zur Energieaufnahme. Theoretisch wäre es möglich, Mittel zur Eingabe aller Mahlzeiten mit den zugehörigen Kalorienwerten vorzusehen. Für den Benutzer ist das allerdings mit grossem Aufwand verbunden, so dass mit Vorteil darauf verzichtet wird. Es ist ausreichend, wenn ein Sollwert S (Fig. 1) für die Energieaufnahme, d.h. ein Wert für die an einem Tag (oder über einen anderen Zeitraum) insgesamt vorgesehene Energieaufnahme programmiert wird. Bevorzugt wird dabei fiktiv von einer kontinuierlichen Aufnahme dieser Energiemenge im Verlaufe des Tages ausgegangen. Mit andern Worten: Für die Berechnung der Energiebilanz zur Stunde x wird die bisherige Energieaufnahme als S/24*x berechnet und dieser Wert mit dem gemessenen, kumulierten Energieverbrauch verglichen.

Die Fiktion einer kontinuierlichen Energieaufnahme hat den zusätzlichen Vorteil, dass sie nicht nur die Berechnung der Energiebilanz seit Beginn der Messperiode sondern auch die Berechnung und Anzeige kurzfristiger Trends dieser Energiebilanz ermöglicht. Es kann jederzeit die Energieaufnahme während einer sich über die letzten n Stunden erstreckenden Analyseperiode als S/24*n berechnet und dieser Wert mit dem für dieselbe Analyseperiode kumulierten Energieverbrauch verglichen werden. Die Länge der Analyseperiode kann dabei durch den Benutzer programmierbar sein und der als Ergebnis ermittelte, kurzfristige Trend der Energiebilanz wird bevorzugt mittels Trendpfeilen 28 auf dem Display 3 angezeigt.

## Patentansprüche

1. Körperaktivitätsmonitor mit einem Speicher 8 und Mitteln (2, 3) zur Eingabe erster Körperdaten eines Benutzers in diesen Speicher, Mitteln zur Messung von einer oder mehreren als Aktivitätsindikatoren dienenden, von der Körperaktivität des Benutzers abhängigen physiologischen Grössen, einer Datenverarbeitungs-Einheit zur Ermittlung des Energieverbrauchs (P) des Benutzers aufgrund der Körperdaten und der Werte der Aktivitätsindikatoren durch das Auswerten einer Energieverbrauchs-Funktion (20) und einem Display (3) zur Anzeige mindestens einer Information zu diesem Energieverbrauch (P), **dadurch gekennzeichnet,**
**dass**
eine Eichungseinheit (15) zur Steuerung des Ablaufs einer Eichungsprozedur vorgesehen ist, durch die bei einer oder mehreren Referenzaktivitäten (R₁, R₂, R₃) des Benutzers die Aktivitätsindikatoren gemessen und von diesen Referenzwerten (T_{R1}, T_{R2}, T_{R3}) eine für die benutzerspezifischen Sensibilität der Aktivitätsindikatoren bezeichnende Indikatorenreferenz abgeleitet und in Ergänzung der ersten Körperdaten gespeichert wird.

2. Körperaktivitätsmonitor nach Anspruch 1, **dadurch gekennzeichnet, dass** die Mittel zur Messung der Aktivitätsindikatoren einen Sensor (9) zur Messung der Herzfrequenz (H) und/oder einen Sensor (11) zur Messung der vertikalen Körperbeschleunigung beinhalten, wobei die gemessene Herzfrequenz bzw. eine von der vertikalen Körperbeschleunigung abgeleitete Grösse (B_{V}) als Aktivitätsindikatoren dienen.

3. Körperaktivitätsmonitor nach einem der Ansprüche 1 oder 2, **dadurch gekennzeichnet, dass** es sich bei den Referenzaktivitäten um mindestens zwei unterschiedliche Intensitätsgrade der Fortbewegung zu Fuss, vorzugsweise des Gehens, handelt.

4. Körperaktivitätsmonitor nach einem der Ansprüche 1 bis 3, **dadurch gekennzeichnet, dass** die Energieverbrauchs-Funktion eine Mehrzahl von Berechnungsformeln (F₁ - F₅) für verschiedene Aktivitätstypen und eine Prozedur (19) zur Aktivitätsklassifizierung beinhaltet, wobei durch diese Prozedur die Aktivität des Benutzers anhand der Werte der Aktivitätsindikatoren und der Indikatorenreferenz einem der Aktivitätstypen zugeordnet und die für diesen vorgesehene Berechnungsformel selektiert wird.

5. Körperaktivitätsmonitor nach Anspruch 4, **dadurch gekennzeichnet, dass** die Energieverbrauchs-Funktion für Werte der Aktivitätsindikatoren, denen die Prozedur (19) zur Aktivitätsklassifizierung keinen bestimmten Aktivitätstyp zuordnet, eine Universalformel für die Berechnung des Energieverbrauchs beinhaltet.

6. Körperaktivitätsmonitor nach Anspruch 4 oder 5, **dadurch gekennzeichnet, dass** den Aktivitätstypen zusammenhängende Bereiche (X₁ - X₅) von Wertekombinationen der Aktivitätsindikatoren zugeordnet sind und die Datenverarbeitungs-Einheit dafür programmiert ist, die Grenzen dieser Indikatorenbereiche (X₁ - X₅) anhand der Indikatorenreferenz zu personalisieren, einen durch die Werte der Aktivitätsindikatoren indizierten, personalisierten Indikatorenbereich (Z₁ - Z₅) zu identifizieren und unter Verwendung der diesem zugehörigen Berechnungsformel (F₁ - F₅) den aktuellen Energieverbrauch zu ermitteln.

7. Körperaktivitätsmonitor nach Anspruch 6, **dadurch gekennzeichnet, dass** die Indikatorenbereiche durch zusammenhängende Bereiche der Wertekombinationen von zwei Aktivitätsindikatoren, vorzugsweise der Herzfrequenz (H) und der von der vertikalen Körperbeschleunigung abgeleiteten Grösse (B_{V}), definiert sind.

8. Körperaktivitätsmonitor nach einem der Ansprüche 6 oder 7, **dadurch gekennzeichnet, dass** die Indikatorenbereiche durch zusammenhängende Bereiche der Wertekombinationen von drei Aktivitätsindikatoren, vorzugsweise der Herzfrequenz (H), einer von der vertikalen Körperbeschleunigung abgeleiteten Grösse (B_{V}) und einer von der horizontalen Körperbschleunigung in Laufrichtung abgeleiteten Grösse (B_{H}) definiert sind.

9. Körperaktivitätsmonitor nach einem der Ansprüche 6 bis 8, **dadurch gekennzeichnet, dass** die Eichungsprozedur (15) die Messung der Aktivitätsindikatoren bei mindestens zwei und vorzugsweise drei Referenzaktivitäten vorsieht und die Indikatorenreferenz die Definition einer Eichkurve (24) in einem durch mindestens zwei Aktivitätsindikatoren aufgespannten Koordinatensystem beinhaltet, die im Wesentlichen durch die von den Referenzwerten (T_{R1}, T_{R2}, T_{R3}) der Aktivitätsindikatoren bezeichneten Punkte verläuft, wobei die Indikatorenbereiche durch Bereiche in diesem Koordinatensystem definiert sind, von denen zumindest einer teilweise durch den Verlauf der Eichkurve (24) definiert ist.

10. Körperaktivitätsmonitor nach Anspruch 9, **dadurch gekennzeichnet, dass** für den Aktivitätstyp der Referenzaktivitäten (R₁, R₂, R₃) eine Formel (F₅) zur Berechnung des Energieverbrauchs vorgesehen ist und die Definition des zur Zuordnung von Aktivitäten zu diesem Aktivitätstyp vorgesehenen Indikatorenbereichs (X₅) einen Toleranzwert (k) für die maximale Abweichung des durch die Aktivitätsindikatoren bezeichneten Punkts von der Eichkurve (24) beinhaltet.

11. Körperaktivitätsmonitor nach einem der Ansprüche 6 bis 8, **dadurch gekennzeichnet, dass** die Indikatorenreferenz einen durch Mittelwerte der Referenzwerte (T_{R1}, T_{R2}, T_{R3}) der Aktivitätsindikatoren bezeichneten Eichpunkt in einem durch mindestens zwei Aktivitätsindikatoren aufgespannten Koordinatensystem beinhaltet, wobei die Indikatorenbereiche durch Bereiche in diesem Koordinatensystem definiert sind, von denen zumindest einer teilweise durch die Lage des Eichpunkts definiert ist.

12. Körperaktivitätsmonitor nach Anspruch 11, **dadurch gekennzeichnet, dass** der Eichpunkt durch die arithmetischen Mittelwerte der Referenzwerte (T_{R1}, T_{R2}, T_{R3}) der Aktivitätsindikatoren definiert ist.

13. Körperaktivitätsmonitor nach Anspruch 11 oder 12, **dadurch gekennzeichnet, dass** einer oder mehrere der Indikatorenbereiche und vorzugsweise sämtliche Indikatorenbereiche eine feste Grösse haben und ihre Lage in dem Koordinatensystem in Bezug auf die Lage des Eichpunkts definiert ist.

14. Körperaktivitätsmonitor nach Anspruch 13, **dadurch gekennzeichnet, dass** die Lagen der Indikatorenbereiche in dem Koordinatensystem durch vorbestimmte Abstände von dem Eichpunkt definiert sind.

15. Körperaktivitätsmonitor nach einem der Ansprüche 1 bis 14, **gekennzeichnet durch** Mittel zur Eingabe des angestrebten Energieverbrauchs (S) und ein Display (3, 28) zur Anzeige der kurzfristigen und/oder der langfristigen Energiebilanz.

16. Körperaktivitätsmonitor nach Anspruch 15, **dadurch gekennzeichnet, dass** die Programmierung eines Werts für die langfristige durchschnittliche Energieaufnahmeleistung (S) vorgesehen und zur Berechnung der Energiebilanz für eine kurze und/oder eine lange Messperiode (x) das Produkt der durchschnittlichen Energieaufnahmeleistung (S) und der Dauer der Messperiode (x) als während dieser Periode (x) getätigte Energieaufnahme angenommen mit dem für dieselbe Periode (x) gemessenen, kumulierten Energieverbrauch verglichen wird.
